(19) Europäisches Patentamt
European Patent Office
Office européen des brevets

(11) **EP 4 700 707 B1**

(12) **EUROPEAN PATENT SPECIFICATION**

(45) Date of publication and mention
of the grant of the patent:
**05.08.2026  Bulletin 2026/32**

(21) Application number: **24201546.9**

(22) Date of filing: **20.09.2024**

(51) International Patent Classification (IPC):
**G06T 12/10** *(2026.01)*

(52) Cooperative Patent Classification (CPC):
**G06T 12/10;** G06T 2211/412

(54) **DR-BASED LARGE-SCALE THREE-DIMENSIONAL IMAGING METHOD AND APPARATUS**

VERFAHREN UND VORRICHTUNG ZUR DR-BASIERTEN DREIDIMENSIONALEN BILDGEBUNG IN GROSSEM MAßSTAB

PROCÉDÉ ET APPAREIL D'IMAGERIE TRIDIMENSIONNELLE À GRANDE ÉCHELLE À BASE DE DR

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **23.08.2024   CN 202411173166**

(43) Date of publication of application:
**25.02.2026   Bulletin 2026/09**

(73) Proprietor: **Shenzhen Angell Technology Co. Ltd.**
**Shenzhen Guangdong 518000 (CN)**

(72) Inventors:
• **YE, Chao**
**SHENZHEN CITY,  GUANGDONG  PROVINCE, 518000 (CN)**
• **ZHENG, Han**
**SHENZHEN  CITY,  GUANGDONG PROVINCE, 518000 (CN)**
• **RONG, Fanzhuang**
**SHENZHEN CITY,  GUANGDONG  PROVINCE, 518000 (CN)**
• **YANG, Jie**
**SHENZHEN CITY,  GUANGDONG  PROVINCE, 518000 (CN)**
• **PI, Zhenzhen**
**SHENZHEN CITY,  GUANGDONG  PROVINCE, 518000 (CN)**

(74) Representative: **Dall'Olio, Christian et al**
**INVENTION S.r.l.**
**Via delle Armi, 1**
**40137 Bologna (IT)**

(56) References cited:
• **BOSSEMA FRANCIEN G ET AL: "Enabling 3D CT-scanning of cultural heritage objects using only in-house 2D X-ray equipment in museums.", NATURE COMMUNICATIONS 14 MAY 2024, vol. 15, no. 1, 14 May 2024 (2024-05-14), pages 3939, XP093257795**

## Description

Technical Field

The present application relates to the field of three-dimensional imaging, in particular to a DR-based large-scale three-dimensional imaging method and apparatus.

Background Art

DR (Digital Radiography) refers to a technology for direct digital radiography under the control of a computer, that is, an X-ray detector converts information of an X-ray penetrating through a human body into a digital signal, and a computer reconstructs an image and performs a series of image post-processing. A DR system mainly consists of several parts, such as an X-ray generation apparatus, an X-ray detector, a system controller, an image monitor, and an image processing workstation.

The development of DR has gone through a plurality of stages including static DR, dynamic DR, and three-dimensional DR. Due to long imaging time of the static DR, images cannot be collected in real time, and static images can be only collected, which cannot satisfy a need of real-time observation for tissue movement and easily causes capturing failure due to the movement of a patient. Later, it developed to the dynamic DR stage. The detector has the capability of collecting the images in real time, can observe and record the tissue movement of the patient in real time, can satisfy cardiopulmonary function observation, joint movement function examination, gastrointestinal contrast examination and other applications so as to have a higher clinical value than the static DR. However, no matter whether it is the static DR or the dynamic DR, only two-dimensional images are obtained. The two-dimensional images cannot avoid interference brought by tissue overlapping. Therefore, in recent years, a few manufacturers have begun to adopt the three-dimensional DR. In the three-dimensional DR, three-dimensional tomographic images are reconstructed by using algorithms through projection images from a plurality of angles, which avoids tissue overlapping, provides precise three-dimensional spatial information, and can accurately obtain information such as a position and size of a lesion, thereby helping doctors more accurately perform diagnosis and treatment. However, the existing three-dimensional DR can only perform three-dimensional imaging on small-scale local tissues due to limitations from a plurality of factors such as a device structure, a scanning way and a detector width so as to be greatly limited in clinical applications. BOSSEMA FRANCIEN G ET AL: "Enabling 3D CT-scanning of cultural heritage objects using only in-house 2D X-ray equipment in museums.", NATURE COMMUNICATIONS 14 MAY 2024, vol. 15, no. 1, discloses a DR (Digital Radiography)-based large scale three-dimensional imaging method according to the preamble of claim 1.

Summary of the Invention

A technical problem to be solved by the present application is to provide a DR-based large-scale three-dimensional imaging method , by which large-scale 3D imaging is performed by DR.

In order to solve the above-mentioned technical problem, the present application discloses a method according to claim 1.

The present application further discloses a DR-based large-scale three-dimensional imaging apparatus for carrying out the method of the invention, including a rack, a detector, a ray generator, a rotation module, a movable supporting base, a patient supporting apparatus, a patient fixing apparatus and a computer terminal; wherein the computer terminal includes a memory, a processor and computer programs stored in the memory and capable of running on the processor, and the processor, when executing the computer programs, implements each step of the above-mentioned DR-based large-scale three-dimensional imaging method of the invention;

the detector and the ray generator are relatively fixed to the rack and are oppositely disposed about the patient supporting apparatus;
the patient supporting apparatus includes a supporting plate and a rotation driving
motor, the rotation driving motor is connected to the supporting plate, and the supporting plate is disposed on the movable supporting apparatus; and
the patient fixing apparatus includes a backup plate and a bandage, one side of the backup plate is fixedly connected to the supporting plate, and one side of the bandage is fixed to the backup plate.

The present application has the beneficial effects that the to-be-processed image captured by the DR device is acquired, the to-be-processed image includes the projection images, and a captured object rotates relative to the ray generator and the detector in a three-dimensional modeling process, the initial capturing angle data acquired by an inductor may have a deviation due to a measurement error or other reasons to affect the precision of the three-dimensional reconstruction, and therefore, the three-dimensional reconstruction can be performed only after the rotation angle is calibrated. In a three-dimensional scenario, a relative position of the same marker to other key points in the captured object should be fixed, by setting markers, the initial capturing angle data of the projection images in the to-be-processed image is calibrated according to the marker feature points in the to-be-processed image after the to-be-processed image is acquired to obtain calibrated initial capturing angles, then, accurate positions of the captured projection images in the three-dimensional space can be determined, and the positions of the large-scale projection images are determined at marked angles in the three-dimensional space by taking the projection images, the calibrated initial capturing angles and the rotation center as parameters in a three-dimensional reconstruction process, and thus, large-scale three-dimensional reconstruction can be achieved.

Brief Description of the Drawings

Fig. 1 is a flow diagram of steps of a DR-based large-scale three-dimensional imaging method in an embodiment of the present application;
Fig. 2 is a schematic diagram of a system cross section coordinate system of a DR-based large-scale three-dimensional imaging apparatus for carrying out the method of the present invention;
Fig. 3 is a schematic diagram of a double-upright-column rack of a DR-based large-scale three-dimensional imaging apparatus for carrying out the method of the present invention;
Fig. 4 is a schematic diagram of a U-shaped arm rack of a DR-based large-scale three-dimensional imaging apparatus for carrying out the method of the present invention;
Fig. 5 is a schematic diagram of a hanging arm rack of a DR-based large-scale three-dimensional imaging apparatus for carrying out the method of the present invention and
Fig. 6 is a schematic diagram of an upright column and hanging arm matched rack of a DR-based large-scale three-dimensional imaging apparatus of the present invention.

Detailed Description of the Invention

In order to describe the technical contents and achieved purposes and effects of the present application in detail, the following description is shown in conjunction with implementations and with cooperation with the accompanying drawings.

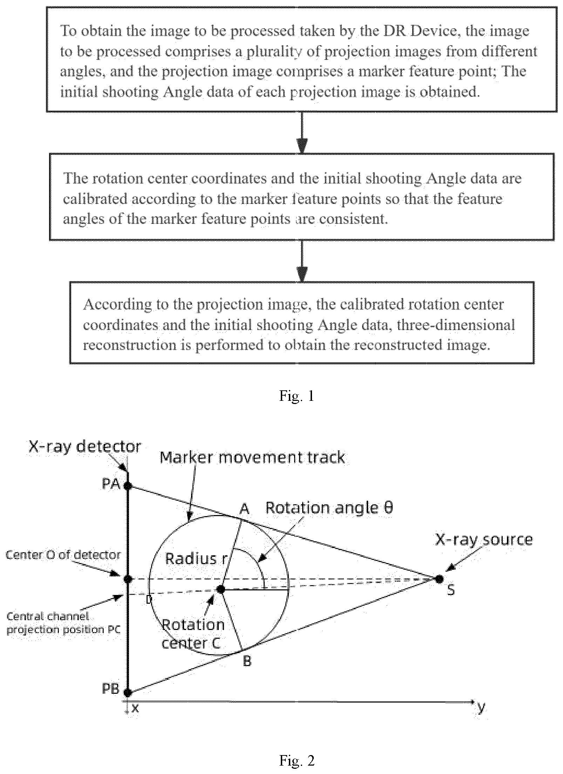

Refer to Fig. 1, provided is a DR-based large-scale three-dimensional imaging method, including the steps:

a to-be-processed image captured by a DR device is acquired, wherein the to-be-processed image includes a plurality of projection images from different angles, and the projection images include marker feature points; initial capturing angle data of each of the projection images is acquired;
coordinates of a rotation center and the initial capturing angle data are calibrated according to the marker feature points so that feature angles of the marker feature points are consistent; and
three-dimensional reconstruction is performed according to the projection images, the calibrated coordinates of the rotation center and the calibrated initial capturing angle data to obtain a reconstructed image.

It can be known from the above-mentioned description that the present application has the beneficial effects that the to-be-processed image captured by the DR device is acquired, the to-be-processed image includes the projection images, and a captured object rotates relative to the ray generator and the detector in a three-dimensional modeling process, the initial capturing angle data acquired by an inductor may have a deviation due to a measurement error or other reasons to affect the precision of the three-dimensional reconstruction, and therefore, the three-dimensional reconstruction can be performed only after the rotation angle is calibrated. In a three-dimensional scenario, a relative position of the same marker to other key points in the captured object should be fixed, by setting markers, the initial capturing angle data of the projection images in the to-be-processed image is calibrated according to the marker feature points in the to-be-processed image after the to-be-processed image is acquired to obtain calibrated initial capturing angles, then, accurate positions of the captured projection images in the three-dimensional space can be determined, and the positions of the large-scale projection images are determined at marked angles in the three-dimensional space by taking the projection images, the calibrated initial capturing angles and the rotation center as parameters in a three-dimensional reconstruction process, and thus, large-scale three-dimensional reconstruction can be achieved.

Further, the step that a to-be-processed image captured by a DR device is acquired includes:

a starting point and an ending point of three-dimensional reconstruction, the maximum single-segment range that can be captured and reconstructed on a single segment by the DR device and an overlapping range between two adjacent segments are acquired;

a total capturing range height is obtained according to the starting point, the ending point and the maximum single-segment range;

a total number of the segments and a movement distance of each of the segments are obtained according to the total capturing range height and the overlapping range;

an actual overlapping range is obtained according to the maximum single-segment range and the movement distance;

a window height is calculated according to the actual overlapping range;

the DR device is adjusted according to the window height, and a window width is set to be the maximum; wherein the window height is calculated along a direction in which the starting point and the ending point are connected; and an exposure starting angle and an exposure ending angle are acquired, and the collection of each of the segments of the to-be-processed image is completed according to the starting point, the exposure starting angle, the exposure ending angle, the movement distance and the ending point.

It can be known from the above-mentioned description that, in a process that the to-be-processed image is acquired, the total capturing range height is obtained according to the preset starting point, ending point and maximum single-segment range that can be captured and reconstructed by the DR device at single time, that is, a part or all of the captured object may be selected, so that the flexibility of the system is enhanced; and the minimum overlapping range is set, data capable of achieving calibration is provided for a subsequent splicing process, the different segments of data can be successfully spliced with a small error, the actual window height is calculated, and finally, the to-be-processed image is acquired according to the calculated parameters, so that the quality of the acquired original to-be-processed image is ensured.

Further, the step that an exposure starting angle and an exposure ending angle are acquired, and the collection of each of the segments of the to-be-processed image is completed according to the starting point, the exposure starting angle, the exposure ending angle, the movement distance and the ending point includes:

exposure is started from the exposure starting angle at the starting point, a rotation module of the DR device is rotated, and the projection images are collected in real time by the DR device;

when the rotation module of the DR device rotates to the exposure ending angle, exposure and the collection of the to-be-processed image are stopped to complete the collection of the current segment of the to-be-processed image; and

a rack of the DR device is moved to the ending point for the movement distance as a new starting point, and the collection of the next segment of the to-be-processed image is continued until the ending point is reached.

It can be known from the above-mentioned description that a projection image can be only acquired by single-time exposure of a ray. Therefore, by rotation, to-be-processed images from a plurality of angles of the same object can be sufficiently acquired, it is convenient to subsequent reconstruction and splicing of three-dimensional images, and the accuracy of the final three-dimensional images is improved; and the movement distance is calculated according to the minimum overlapping range, it is ensured that splicing reference can be acquired according to the overlapping range in a splicing process, the accuracy of a splicing result is further ensured, radiation received by the object is reduced, and the health of a patient is further ensured during three-dimensional reconstruction for the patient.

Further, the step that coordinates of a rotation center and the initial capturing angle data are calibrated according to the marker feature points includes:

a central point O of a detector of the DR device, an X-ray source point S and initial capturing angle data $\theta_i$ of the $i^{th}$ projection image are acquired;

a first distance SID between the central point of the detector and the X-ray source point is calculated;

a two-dimensional rectangular coordinate system is established by taking a plane where the detector is located as an x axis and a line parallel to a straight line where a connecting line between the ray source point and the central point of the detector is located as a y axis; a projection position $(px_i, 0)$ of the marker feature point in the projection image on the plane where the detector is located is obtained;

the maximum projection position $(px_A, 0)$ of a first projection image with the maximum $px_i$ value is acquired, corresponding first measurement angle data $\theta_A = \text{actan}(SID/px_A)$ is calculated according to the maximum projection position, first initial capturing angle data of the first projection image is acquired, and a first difference $d\theta_A$ of the first measurement angle data and the first initial capturing angle data is obtained;

the minimum projection position $(px_B, 0)$ of a second projection image with the minimum $px_i$ value is acquired, corresponding second measurement angle data $\theta_B = \text{actan}(SID/px_B)$ is calculated according to the minimum projection position, second initial capturing angle data of the second projection image is acquired, and a second difference

$d\theta_B$ of the second measurement angle data and the second initial capturing angle data is obtained;

a third difference of x coordinates of two adjacent projection images is calculated, the maximum difference projection position ($px_D$, 0) of the third difference and a corresponding third projection image are acquired, corresponding third measurement angle data $\theta_D$=actan(SID/$px_D$) is calculated according to the difference projection position, third initial capturing angle data of the corresponding third projection image is acquired, and a third difference $d\theta_D$ of the third measurement angle data and the third initial capturing angle data is obtained;

an average angle deviation $d\theta$ is obtained according to the first difference, the second difference and the third difference; and

each piece of the initial capturing angle data $\theta_i$ is corrected according to the average angle deviation.

It can be known from the above-mentioned description that, before rotation and marking, firstly, an error value of an angle obtained by measurement is marked according to a feature point that can be acquired, and a measured initial capturing angle value is corrected according to a marking result to ensure the precision of subsequent calculation.

Further, the step that three-dimensional reconstruction is performed according to the projection images, the calibrated coordinates of the rotation center and the calibrated initial capturing angle data to obtain a reconstructed image includes:

the projection images, calibrated initial capturing angles corresponding to the projection images and a calibrated rotation center are input to a reconstruction module to perform three-dimensional reconstruction to obtain the reconstructed image.

It can be known from the above-mentioned description that the special reconstruction module is disposed, a reconstruction process for the three-dimensional images is performed by the reconstruction module, three-dimensional reconstruction is performed according to the projection images and the calibrated data to further improve the precision of the reconstructed image, and the special reconstruction module is convenient to manage.

Further, the step that coordinates of a rotation center and the initial capturing angle data are calibrated according to the marker feature points further includes:

a first expression $x_A$=r*cos($\theta_A$)+$x_C$, $y_A$=r*sin($\theta_A$)+$y_C$ of coordinates of a first origin A corresponding to the maximum projection position ($px_A$, 0) is constructed;

a second expression $x_B$=r*cos($\theta_B$)+$x_C$, $y_B$=r*sin($\theta_B$)+$y_C$ of coordinates of a second origin B corresponding to the minimum projection position ($px_B$, 0) is constructed;

a third expression $x_D$=r*cos($\theta_D$)+$x_C$, $y_D$=r*sin($\theta_D$)+$y_C$ of coordinates of a third origin D corresponding to the difference projection position ($px_D$, 0) is constructed;

a projection mapping formula px*(SID-y)=x*SID is acquired;

wherein px represents coordinates of a projected x axis, (x, y) represents coordinates of an origin before projection, and the origin includes the first origin, the second origin and the third origin; and

a solution is sought according to the first expression, the second expression, the third expression and the projection mapping formula to obtain coordinates ($x_C$, $y_C$) of the rotation center and a rotation radius r.

It can be known from the above-mentioned description that the coordinates of the rotation center is determined after the coordinates of the feature points are calculated, then, three-dimensional reconstruction can be subsequently performed according to the calibrated rotation center, and thus, the calculation precision is further improved.

Further, the step that three-dimensional reconstruction is performed according to the projection images, the calibrated coordinates of the rotation center and the calibrated initial capturing angle data to obtain a reconstructed image includes:

three-dimensional reconstruction is performed according to the projection image in each of the segments, the calibrated coordinates of the rotation center and the calibrated initial capturing angle data to obtain three-dimensional images;

the three-dimensional images corresponding to the segments are traversed, when target three-dimensional images are traversed, intermediate tomographic images in the target three-dimensional images are acquired, marker feature points in the intermediate tomographic images are acquired, the current correction angle of a connecting line between each marker feature point and the rotation center relative to the straight line where the connecting line between the ray source point and the central point of the detector is located is calculated, and the current correction angle is compared with a historical correction angle of a previous three-dimensional image, if the current correction angle is inconsistent with the historical correction angle, the three-dimensional images are rotated so that the current correction angle is consistent with the historical correction angle; corrected target three-dimensional images are obtained; and

all the corrected target three-dimensional images are spliced to obtain the reconstructed image.

It can be known from the above-mentioned description that an angle between the three-dimensional images obtained by reconstructing two adjacent segments is further aligned after three-dimensional reconstruction is performed on each segment to obtain the three-dimensional images, so that angle errors caused in the splicing process of the reconstructed different segments of the three-dimensional images are avoided, and the precision of the final reconstructed image is further ensured.

Further, the step that all the corrected target three-dimensional images are spliced to obtain the reconstructed image includes:

the segments are traversed, when targets segments are traversed, the corrected target three-dimensional images corresponding to the target segments and a position (x0, y0, z0) of a historical comparison area of a previous target segment are acquired; the current comparison area with the highest similarity is searched in target tomographic images of the corrected target three-dimensional images according to a content of the historical comparison area, and a position (x1, y1, z1) of the current comparison area is recorded; a deviation value $\Delta d=(x0, y0, z0)-(x1, y1, z1)$ of the current comparison area relative to the historical comparison area is calculated; and the corrected target three-dimensional images are translated according to the deviation value; and

the target tomographic images in the corrected target three-dimensional images are acquired, a new historical comparison area is acquired within an overlapping range of the corrected target three-dimensional images, and a position of the historical comparison area is updated.

It can be known from the above-mentioned description that the deviations of the target three-dimensional images corresponding to the segments are corrected one by one by traversing, so that errors are reduced, and the subsequent splicing effect is ensured.

Further, after the step that the DR device is adjusted according to the window height, and a window width is set to be the maximum, the DR-based large-scale three-dimensional imaging method includes:

exposure is started from the exposure starting angle at the starting point, and the rotation module of the DR device is rotated, at the same time, the DR device is moved to the ending point, and the projection images, the initial capturing angle data corresponding to the projection images and a movement position of the rack are collected in real time by the DR device; and

when the DR device reaches the ending point, and the rotation module rotates to the exposure ending angle, exposure and the collection of the to-be-processed image are stopped; and

the step that three-dimensional reconstruction is performed according to the projection images, the calibrated coordinates of the rotation center and the calibrated initial capturing angle data to obtain a reconstructed image includes:

the movement position of the rack, the projection images, calibrated initial capturing angles corresponding to the projection images and the calibrated coordinates of the rotation center are input to a reconstruction module to perform three-dimensional reconstruction to obtain the reconstructed image.

It can be known from the above-mentioned description that provided is another image collection method in which the rack also moves from the starting point to the ending point while rotating, by which one-step reconstruction in place can be achieved during reconstruction. Compared with a way of segmented reconstruction and then splicing, the method lies in that the splicing step is omitted, one-step reconstruction can be completed only by inputting the movement position of the rack as a parameter in the reconstruction process to the reconstruction module, and types of errors that may appear can also be reduced due to the reduction of the step. Further, the rack may be set to move at a constant speed, the movement position of the rack can be precisely obtained according to the speed and time of the constant-speed movement of the rack, and thus, the precision of the reconstructed image is further ensured.

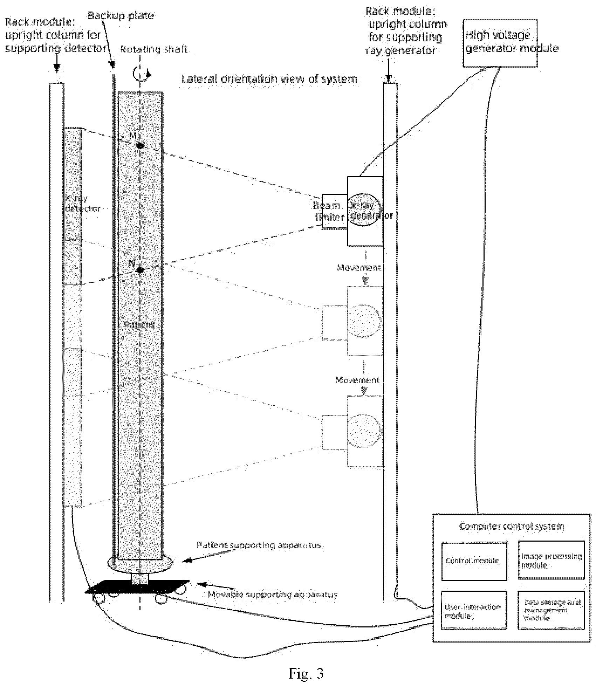

Refer to Fig. 3 to Fig. 6, provided is a DR-based large-scale three-dimensional imaging apparatus for carrying out the method of the invention, including a rack, a detector, a ray generator, a rotation module, a movable supporting base, a patient supporting apparatus, a patient fixing apparatus and a computer terminal; wherein the computer terminal includes a memory, a processor and computer programs stored in the memory and capable of running on the processor, and the processor, when executing the computer programs, implements each step of the above-mentioned DR-based large-scale three-dimensional imaging method of the invention;

the detector and the ray generator are relatively fixed to the rack and are oppositely disposed about the patient supporting apparatus;

the patient supporting apparatus includes a supporting plate and a rotation driving motor, the rotation driving motor is connected to the supporting plate, and the supporting plate is disposed on the movable supporting apparatus; and

the patient fixing apparatus includes a backup plate and a bandage, one side of the backup plate is fixedly connected to the supporting plate, and one side of the bandage is fixed to the backup plate.

It can be known from the above-mentioned description that the rack supports the detector and the ray generator which are oppositely disposed about the patient supporting apparatus, and thus, a ray emitted by the ray generator may be received by the detector; moreover, the patient supporting apparatus is provided with the rotation driving motor, and thus, the patient supporting apparatus may rotate under the drive of a rotation motor to acquire a to-be-processed image; and the computer terminal may control the apparatus according to the above-mentioned DR-based large-scale three-dimensional imaging method; that is, provided is the apparatus capable of implementing the above-mentioned DR-based large-scale three-dimensional imaging method of the invention.

The above-mentioned DR-based large-scale three-dimensional imaging method and apparatus for carrying out the method of the present invention are applicable to the field in which large-scale three-dimensional imaging is required, for example, the whole bodies of patients need to be scanned in hospitals, and a more accurate and effective imaging method is provided for clinical applications, such as the examination for whole body postures, whole spine deformity and whole lower limb deformity. They have important clinical significance, which will be described as follows with specific implementations.

Refer to Fig. 1 to Fig. 2, embodiment 1 of the method of present invention is that: provided is a DR-based large-scale three-dimensional imaging method, including the steps:

S1, a to-be-processed image captured by a DR device is acquired, wherein the to-be-processed image includes a plurality of projection images from different angles, and the projection images includes marker feature points; initial capturing angle data of each of the projection images is acquired, which includes:

S10, system reset: a control module controls a supporting apparatus to return to a 0-degree point. A control method includes: a 0-degree detector signal is detected, when the 0-degree detector signal is not detected, the supporting apparatus is controlled to rotate until the 0-degree detector signal is detected, and then, rotation is stopped; and the 0-degree point is a point where markers are closest to a detector;

starting and ending point selection: the detector and a ray generator are moved to a starting point of a capturing range by a movement control rod, and the starting point is determined by a starting point selection key; and the detector and the ray generator are moved to an ending point of the capturing range by the movement control rod, and the ending point is determined by an ending point selection key;

exposure condition setting: the control module sets filtration according to an age and weight of a patient, sets filtration for a child patient to reduce radiation injury of soft rays to children; and high-voltage voltage and current values are set; and

if a to-be-processed image of the patient is collected, information registration is further included: patient information is input for registration;

S11, a starting point Q and an ending point E of three-dimensional reconstruction, the maximum single-segment range Z that can be captured and reconstructed at single time by the DR device, and an overlapping range c between two adjacent segments are acquired; the overlapping range is the minimum overlapping range required in a splicing process;

S12, a total capturing range height H=Z+E-Q is obtained according to the starting point, the ending point and the maximum single-segment range;

S13, a total number N of the segments and a movement distance d of each of the segments according to the total capturing range height and the minimum overlapping range;

N=ceil(H/(Z-c)), wherein ceil represents that the maximum and closest integer is taken;

$$d=(H-Z)/(N-1);$$

S14, an actual overlapping range c'=Z-d is obtained according to the maximum single-segment range and the movement distance;

an ideal state is that a movement error is reduced as much as possible, and radiation is performed as less as possible except that it is ensured that a sufficient overlapping range required in the splicing process is reserved;

S15, a window height is calculated according to the actual overlapping range;

if the overlapping range is overwide, the patient will accept excessive useless rays, and therefore, a window height of a collimator needs to be reduced to shield unnecessary rays, the window height on the position of the rotation center is set as h=Z-c'+c, a single-segment actual imaging range is set as Z'=h, and the window width is the maximum, so that the maximum width is obtained in a transverse direction;

since an X-ray is a conical beam, on the premise that an opening of the collimator is unchanged, the longer the distance away from an X-ray light source point is, the wider the irradiation range (window) of light is, and the window height described herein refers to the window height calculated on the position of the rotation center, i.e., a height of MN in Fig. 3;

S16, the DR device is adjusted according to the window height, and a window width is set to be the maximum; wherein the window height is calculated along a direction in which the starting point and the ending point are connected;

S17, an exposure starting angle and an exposure ending angle are acquired, and the collection of each of the segments of the to-be-processed image is completed according to the starting point, the exposure starting angle, the exposure ending angle, the movement distance and the ending point;

S2, coordinates of a rotation center and the initial capturing angle data are calibrated according to the marker feature points so that feature angles of the marker feature points are consistent;

refer to Fig. 2, it can be seen that, due to device setting, if a central point O of the detector, a ray source point S and a rotation center C are not located on the same horizontal line, errors may be generated on a projection PC of the rotation center and the center O of the detector, that is, there are errors between a measured rotation angle $\theta$ and an actual rotation angle, and thus, an angle calibration step includes:

S201, a central point O of a detector of the DR device, an X-ray source point S and initial capturing angle data $\theta_i$ of the $i^{th}$ projection image are acquired;

S202, a first distance SID between the central point of the detector and the X-ray source point is calculated;

S203, a two-dimensional rectangular coordinate system is established by taking a plane where the detector is located as an x axis and a line parallel to a straight line where a connecting line between the ray source point and the central point of the detector is located as a y axis; a projection position $(px_i, 0)$ of the marker feature point in the projection image on the plane where the detector is located is obtained;

in an optional implementation, markers adopt objects having obvious features in a ray scanning process so as to have obvious edge signals, positions (px, py) of the markers in the projection images may be obtained by edge extraction, and thus, it can be known according to the established coordinate system that the plane where the detector is located as the x axis, then, py=0, and thus, $(px_{ij}, 0)$ may be obtained according to each frame of image, i.e., the captured projection images;

S204, the maximum projection position $(px_A, 0)$ of a first projection image with the maximum $px_i$ value is acquired, corresponding first measurement angle data $\theta_A=actan(SID/px_A)$ is calculated according to the maximum projection position, first initial capturing angle data of the first projection image is acquired, and a first difference $d\theta_A$ of the first measurement angle data and the first initial capturing angle data is obtained;

S205, the minimum projection position $(px_B, 0)$ of a second projection image with the minimum $px_i$ value is acquired, corresponding second measurement angle data $\theta_B=actan(SID/px_B)$ is calculated according to the minimum projection position, second initial capturing angle data of the second projection image is acquired, and a second difference $d\theta_B$ of the second measurement angle data and the second initial capturing angle data is obtained;

wherein the maximum projection position and the minimum projection position refer to positions of two projection points PA and PB farthest from the center O of the detector;

S206, a third difference of x coordinates of two adjacent projection images is calculated, the maximum difference projection position $(px_D, 0)$ of the third difference and a corresponding third projection image are acquired, corresponding third measurement angle data $\theta_D=actan(SID/px_D)$ is calculated according to the difference projection position, third initial capturing angle data of the corresponding third projection image is acquired, and a third difference $d\theta_D$ of the third measurement angle data and the third initial capturing angle data is obtained;

S207, an average angle deviation $d\theta=(d\theta_A+d\theta_B+d\theta_D)/3$ is obtained according to the first difference, the second difference and the third difference; and

S208, each piece of the initial capturing angle data $\theta_i=\theta_i+d\theta$ is corrected according to the average angle deviation;

in an optional implementation, the step further includes:

S209, a first expression $x_A=r*cos(\theta_A)+x_C$, $y_A=r*sin(\theta_A)+y_C$ of coordinates of a first origin A corresponding to the maximum projection position $(px_A, 0)$ is constructed;

a second expression $x_B=r*cos(\theta_B)+x_C$, $y_B=r*sin(\theta_B)+y_C$ of coordinates of a second origin B corresponding to the minimum projection position $(px_B, 0)$ is constructed;

a third expression $x_D=r*cos(\theta_D)+x_C$, $y_D=r*sin(\theta_D)+y_C$ of coordinates of a third origin D corresponding to the difference projection position $(px_D, 0)$ is constructed;

a projection mapping formula $px*(SID-y)=x*SID$ is acquired;

wherein px represents coordinates of a projected x axis, (x, y) represents coordinates of an origin before projection, and the origin includes the first origin, the second origin and the third origin; and

S210, a solution is sought according to the first expression, the second expression, the third expression and the projection mapping formula to obtain coordinates $(x_C, y_C)$ of the rotation center and a rotation radius r;

refer to Fig. 2, it can be known that original marker feature points A and B corresponding to the projection points PA and PB farthest from the center of the detector are tangent points on a marker movement track, and therefore, it may be determined that $\angle PA$ is the same as $\angle ACS$, and a rotation angle $\theta$, i.e., an actual rotation angle, may be obtained by calculating LPA; at the same time, it is set that a starting position of angle calculation is parallel to a connecting line of SO, the y axis is also parallel to the connecting line of SO, and it can be known that the speed of point D farthest from an initial position is highest when rotation is stopped at the initial position after rotation is performed for a cycle, and therefore, point D is affirmed by affirming the maximum speed, a rotation angle corresponding to point D is solved by solving ∠OSPC, the three points are determined in such a way, and thus, the rotation center is determined;

for example, the markers are set as metal wires or small metal balls uniformly distributed from top to bottom, and thus, after three-dimensional reconstruction is performed according to the calibrated initial capturing angle data and the rotation center, the marker feature points are also located on the same straight line in the reconstructed image; and the marker feature points are used as reference points for angle calibration, so that corresponding positions in a three-dimensional space may be determined according to other points on the projection images rotating with the marker feature points. In an optional implementation, all the projection images may be rotated for the initial capturing angles to return to a 0-degree position in the reconstruction process;

S3, three-dimensional reconstruction is performed according to the projection images, the calibrated coordinates of the rotation center and the calibrated initial capturing angle data to obtain a reconstructed image, which includes:

the projection images, calibrated initial capturing angles corresponding to the projection images and a calibrated rotation center are input to a reconstruction module to perform three-dimensional reconstruction to obtain the reconstructed image.

Embodiment 2 of the method of the present invention is that:

provided is a DR-based large-scale three-dimensional imaging method different from embodiment 1 in that S17 includes:

S171, exposure is started from the exposure starting angle at the starting point, a rotation module of the DR device is rotated, and the projection images are collected in real time by the DR device;

in an optional implementation, the rotation module of the DR device is rotated at a constant speed;

S172, when the rotation module of the DR device rotates to the exposure ending angle, exposure and the collection of the to-be-processed image are stopped to complete the collection of the current segment of the to-be-processed image; and

S173, a rack of the DR device is moved to the ending point for the movement distance as a new starting point, and the collection of the next segment of the to-be-processed image is continued until the ending point is reached;

in a movement process, rotation may be stopped, or the collection of the to-be-processed image is only stopped, and if the rotation is not stopped, it is unnecessary to turn on or off the rotation module all the time; when the rotation is stopped, the rotation may be stopped at the 0-degree position, and thus, when the next segment of the to-be-processed image is collected, it is unnecessary to calibrate the 0-degree position, and the rack may move to a corresponding position according to the starting angle to start the collection;

wherein each segment of the to-be-processed image represents images acquired within the same height range, and the to-be-processed image includes a plurality of projection images collected in a process that the DR rotates from the exposure starting angle to the exposure ending angle within the height range;

in an optional implementation, the step further includes steps that: the to-be-processed image is pre-processed; the collected to-be-processed image is primarily processed such as bad point correction, de-noising and brightness conversion; bad point correction is to fill up bad pixels according to a mean or interpolation of normal pixels surrounding a bad pixel point; de-noising may be performed by using a common de-noising method, such as a Gaussian filter, a mean filter and a median filter; and brightness conversion is to convert the brightness of an image into a relationship directly proportional to an attenuation rate, with a conversion relationship being I'=log(I0/I), wherein I0 represents an image collected in advance and captured when there is no patient, I represents an image captured when there is a patient, i.e., the to-be-processed image, and I' represents a converted image;

S3, three-dimensional reconstruction is performed according to the projection images, the calibrated coordinates of the rotation center and the calibrated initial capturing angle data to obtain a reconstructed image, which includes:

S31, three-dimensional reconstruction is performed according to the projection image in each of the segments, the calibrated coordinates of the rotation center and the calibrated initial capturing angle data to obtain three-dimensional images;

wherein a reconstruction algorithm may be an analytic algorithm based on filtered back-projection or an iterative algorithm based on algebraic equations; the reconstruction algorithm is used for reconstructing three-dimensional images within an effective exposure range according to a size of a beam limiter and removing a shielding part of the beam limiter;

S32, the three-dimensional images corresponding to the segments are traversed, when target three-dimensional images are traversed, intermediate tomographic images in the target three-dimensional images are acquired, marker feature points in the intermediate tomographic images are acquired, the current correction angle of a connecting line between each marker feature point and the rotation center relative to the straight line where the connecting line between the ray source point and the central point of the detector is located is calculated, and the current correction angle is compared with a historical correction angle of a previous three-dimensional image, if the current correction angle is inconsistent with the historical correction angle, the three-dimensional images are rotated so that the current correction angle is consistent with the historical correction angle; corrected target three-dimensional images are obtained;

the tomographic images are horizontal plane slice images in the three-dimensional reconstructed image and are equivalent to information seen after the image is horizontally cut once; and the intermediate tomographic images are tomographic images on an intermediate height of a segment of reconstructed three-dimensional image;

S33, all the corrected target three-dimensional images are spliced to obtain the reconstructed image, which includes:

S331, the segments are traversed, when target segments are traversed, the corrected target three-dimensional images corresponding to the target segments and a position (x0, y0, z0) of a historical comparison area M0 of a previous target segment are acquired;

S332, the current comparison area M1 with the highest similarity is searched in target tomographic images of the corrected target three-dimensional images according to a content of the historical comparison area, and a position (x1, y1, z1) of the current comparison area is recorded;

in an optional implementation, a similarity calculation method is that: the similarity $R=\Sigma(M1(u,v,k)-M0(u,v,k))2$, wherein (u, v, k) represents a pixel coordinate value in a comparison area (including the historical comparison area and the current comparison area); and M1(u,v,k) represent a gray value on point (u, v, k);

in an optional implementation, all the target tomographic images of the calibrated target three-dimensional images are acquired, the current comparison area M1 with the maximum similarity to M0 is started to be retrieved layer by layer from a first layer of tomographic image within an overlapping range within which the target tomographic image corresponds to the historical comparison area, and the position of the current comparison area is recorded;

S33, a deviation value $\Delta d=(x0, y0, z0)-(x1, y1, z1)$ of the current comparison area relative to the historical comparison area is calculated; and the target three-dimensional images are translated according to the deviation value;

specifically, $\Delta x==x0-x1$, $\Delta y=y0-y1$, $\Delta z=z0-z1$, and the corrected target three-dimensional images are moved respectively from the x axis, the y axis and the z axis according to the calculation result; a way of establishing a coordinate axis is not limited herein as long as a position of each of the feature points in the three-dimensional images may be marked;

wherein the translation of the three-dimensional images means that a gray value of a coordinate pixel in the current target three-dimensional image is moved to a pixel of a target coordinate according to the deviation value;

a range value of a z coordinate in the overlapping area is $z \in (Z-\Delta z, Z)$; wherein Z represents the maximum single-segment range that can be captured and reconstructed at single time by the DR device; the deviation value has been corrected, and therefore, the corrected coordinate is an accurate value; and the range of the z coordinate in the overlapping range is calculated by using the deviation value herein;

S34, the target tomographic images in the corrected target three-dimensional images are acquired, a new historical comparison area is acquired within an overlapping range of the target tomographic images, a position of the historical comparison area is updated, and then, S31 is further performed;

that is, for a segmented collection way, each segment of the three-dimensional images reconstructed after angles of the projection images are calibrated may be directly spliced, that is, S31 and S33 are performed, if it is expected to further improve the precision, S32 may be additionally performed; and a possible angle deviation between two adjacent segments are further aligned;

the overlapping range in S3 may be the maximum overlapping range or the minimum overlapping range which may be selected according to a specific running demand, the maximum overlapping range may avoid omission, and the minimum overlapping range may accelerate the calculation efficiency; the minimum overlapping range is $cmin=Z-d-e$; the maximum overlapping range is $cmax=Z-d+e$; wherein d represents a movement distance of the rack when two adjacent segments of the to-be-processed image are acquired, and e represents a movement error of the rack;

after the corrected target three-dimensional images are obtained by three-dimensional reconstruction, a marker is a point on a horizontal slice image, an angle between a connecting line of the point and the rotation center and the y axis or a direction of the marker is different in different data segments, the direction needs to be adjusted to be consistent, and then, splicing may be performed; and specifically, the same point may be imaged in an overlapping area of two adjacent segments, if there is a difference in angles, the same point of two segment of data does not overlap after splicing, and therefore, the angles need to be adjusted to be the same to overlap, and thus, splicing is achieved;

S33 further includes:

weighted fusion is performed on the overlapping range; and specifically, weighted fusion is performed on the tomographic images that have been marked as the same positions (M0 and M1) in the two adjacent segments so that data between the two segments is in smooth translation;

$$I=(1-w)*I0(x,y,z)+w*I1(x,y,z);$$

wherein I represents a fusion result; I0 represents an adjacent first segment of to-be-fused data, I1 represents an adjacent second segment of to-be-fused data, $z \in (Z-d, Z)$, a weighted value is expressed as $w=(z-Z+d)/d$, z represents a longitudinal coordinate within the overlapping range; that is, data, closer to the second segment of data, in the first segment of data has a higher weight, weighted fusion is performed on the overlapping range in the splicing process, so that features in two target to-be-processed images may be taken into account within the overlapping range, and then, the splicing effect is further improved.

Embodiment 3 of the method of the present invention is that:

provided is a DR-based large-scale three-dimensional imaging method different from embodiment 1 or embodiment 2 in that after S16, the DR-based large-scale three-dimensional imaging method includes:

S171, exposure is started from the exposure starting angle at the starting point, and the rotation module of the DR device is rotated, at the same time, the DR device is moved to the ending point, and the projection images, the initial capturing angle data corresponding to the projection images and a movement position of the rack are collected in real time by the DR device;

in an optional implementation, the rack is moved by constant-speed rotation or movement;

S172, when the DR device reaches the ending point, and the rotation module rotates to the exposure ending angle, exposure and the collection of the to-be-processed image are stopped;

S3 includes: the movement position of the rack, the projection images, calibrated initial capturing angles corresponding to the projection images and the calibrated coordinates of the rotation center are input to a reconstruction module to perform three-dimensional reconstruction to obtain the reconstructed image;

for a way of rotating while moving, the position of the rack may be precisely obtained according to a speed and a time under the condition that the rack moves at a constant speed, two-dimensional images, the corrected rotation center, the corrected angle data and the precise position of the rack are directly input to the reconstruction module to perform three--dimensional reconstruction, and thus, the reconstruction precision is ensured.

Refer to Fig. 3 to Fig. 6, embodiment 4 of the method of the present invention is that:

provided is a DR-based large-scale three-dimensional imaging apparatus for carrying out the method of the invention, including a rack, a detector, a ray generator, a movable supporting base, a patient supporting apparatus, a patient fixing apparatus and a computer terminal; wherein the computer terminal includes a memory, a processor and computer programs stored in the memory and capable of running on the processor, and the processor, when executing the computer programs, implements each step of the DR-based large-scale three-dimensional imaging method in embodiment 1;

the detector and the ray generator are relatively fixed to the rack and are oppositely disposed about the patient supporting apparatus; and in an optional implementation, the detector is closer to the patient supporting apparatus than the ray generator;

refer to Fig. 3, in an optional implementation, the rack is of a double-upright-column structure, the detector and the ray generator are respectively supported by two upright columns perpendicularly fixed on the ground, and the detector and the ray generator may synchronously move up and down along the upright columns to acquire different segments of the to-be-processed image. A rotation module is disposed between the detector and the ray generator and is close to the detector;

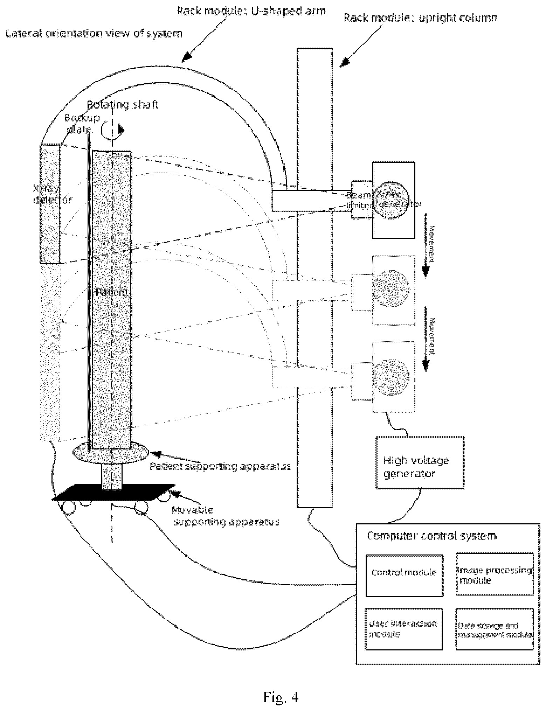

refer to Fig. 4, in an optional implementation, the rack includes a U-shaped arm and an upright column, one end of the U-shaped arm is movably connected to the upright column fixed to the ground and is provided with the ray generator, the other end of the U-shaped arm is provided with the detector, and the U-shaped arm moves up and down relative to the upright column in a movement process, thereby acquiring different segments of the to-be-processed image;

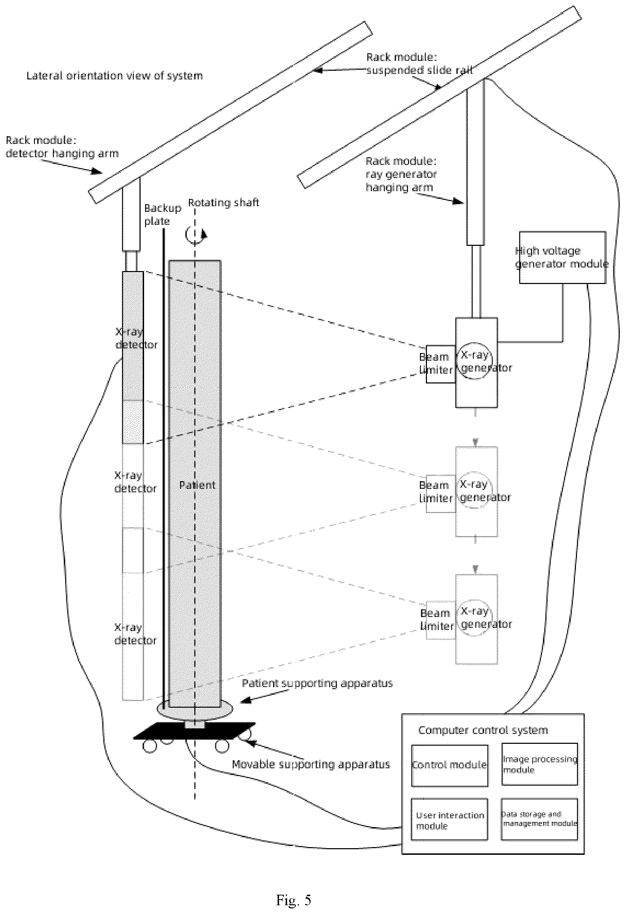

refer to Fig. 5, in an optional implementation, the rack is of a double-hanging-arm structure; the detector and the ray generator are respectively fixed to two hanging arms, the hanging arms are of telescopic bucket structures, the detector and the ray generator are controlled to synchronously move upwards or downwards by telescoping, and a rotation module is disposed between the detector and the ray generator and is close to the detector; and the hanging arms may be fixed to a ceiling of a room where the device is arranged, for example, the ceiling may also be provided with orbits matched with the hanging arms, and thus, more flexible adjustment may be achieved according to the position of a to-be-collected object and a part required to be collected;

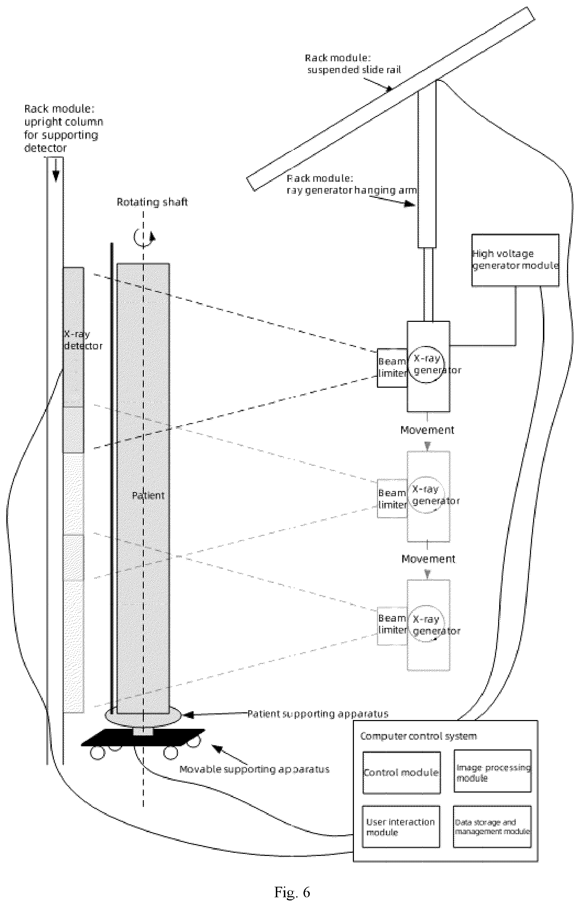

refer to Fig. 6, in an optional implementation, the rack is of a hanging arm and upright column matched structure, the detector may be disposed on an upright column, and the ray generator may be disposed on a hanging arm; or the detector is disposed on the hanging arm, the ray generator is disposed on the upright column, and a rotation module is disposed between the detector and the ray generator;

in an optional implementation, the detector is an X-ray detector, the ray generator is an X-ray generator, other rays capable of acquiring the projection images may also be used as required;

the ray generator directly faces the detector and is spaced from the detector for a certain distance, the ray generator and the detector of the rack may be controlled by a control module to synchronously move upwards or downwards to a specified height position, so that a system may perform exposure and collection at different heights. Since the width of the detector is limited, an image of a certain local tissue of a patient may be only obtained by capturing a fixed position, and the imaging range is limited; however, if capturing is performed by moving the detector and the ray generator up and down to splice images of different positions together, a wider longitudinal imaging range may be obtained, for example, the whole body of the patient is divided into a plurality of segments to be captured, and then, the plurality of segments of images are spliced together by using a splicing algorithm to obtain an image of the whole body;

the rotation module is used for supporting and fixing a patient and driving the patient to rotate, so that the system may capture images of the patient from different angles to further perform three-dimensional reconstruction. The rotation module includes apparatuses such as a movable supporting base, a patient supporting apparatus, a patient fixing apparatus, an angle measurement and synchronization apparatus and a geometric correction apparatus;

the patient supporting apparatus includes a supporting plate and a rotation driving motor, the rotation driving motor is connected to the supporting plate, and the supporting plate is disposed on the movable supporting apparatus; the patient stands on the supporting plate, the center of the supporting plate overlaps with the center of a rotating shaft, the supporting plate is driven by a driving motor to rotate, and then, the patient is driven to rotate. The driving motor may rotate at a certain constant speed, and the starting, stopping and rotating speeds of the motor are controlled by the control module;

the movable supporting base may flexibly move to satisfy different capturing demands. The bottom of the movable supporting base is provided with four universal wheels that may be locked to avoid moving when being required to be fixed; during three-dimensional scanning, the rotation module is wholly moved between the detector and the ray generator, and the center of the detector, a rotation center and a ray center are aligned in the same straight line as much as possible. In order to reduce an amplification effect, the rotation module is placed on a position close to the detector, and thus, a wider transverse imaging range may be obtained. During two-dimensional capturing, the rotation module may be removed to achieve conventional two-dimensional DR capturing. In order to ensure the position consistence every time, a positioning apparatus is mounted on the ground to ensure that the rotation module may be restored to the same position after movement every time;

the patient fixing apparatus includes a backup plate and a bandage, one side of the backup plate is fixedly connected to the supporting plate, and one side of the bandage is fixed to the backup plate; and the patient clings to a bed board in a rotation process and is bound and fixed to the bed board by using the bandage, which ensures that the body of the patient keeps still in a movement process;

a high voltage and ray generation module composed of a high voltage generator, a ray generator and a beam limiter is further included. The high voltage generator is used for generating a high voltage that may be loaded to the ray generator to generate an X-ray, and the production of the X-ray may be controlled to be started or stopped by the control module. The beam limiter is mainly composed of a beam limiting window and a filter. The beam limiting window may limit the generated X-ray within a certain window range to avoid the diffusion of the radiation of the X-ray to the surrounding, and at the same time, a ray window range may be controlled by the control module to achieve different-width irradiation imaging. The filter is used for filtering soft rays incapable of penetrating through a human body to reduce unnecessary radiation, filters with a plurality of thicknesses may be selected, the filters with the different thicknesses may be switched by the control module, and the material of the filter includes, but is not limited to metal materials, such as copper and aluminum;

a data collection and transmission module includes a detector and a transmission module. The detector is used for collecting an X-ray signal and converting the same into image data, a detector with a dynamic function is adopted so that image data in a movement process of a patient or an object may be collected rapidly in real time, and the detector may be controlled by the control module to start or stop collection. The transmission module is used for transmitting the image data from the detector to a data storage and management module for storage in real time through a high-bandwidth network card on the basis of a protocol. The transmission module is used for dividing the data into a plurality of data packets with serial numbers and transmitting the data on the basis of a UDP protocol which may cause data loss, and when a data packet with a certain serial number is missed, it is determined as packet loss, and the interpolation of the lost data packet is repaired by using an adjacent data packet;

the angle measurement and synchronization apparatus is used for measuring a rotation angle at each image collection time and feeding the same back to an image processing module to perform three-dimensional reconstruction, and includes an angle synchronization apparatus and an angle measurement apparatus. The angle synchronization apparatus is used for determining a 0-degree rotation position, synchronously performing angle measurement at each image collection time to make angle data correspond to images one by one, and includes a 0-degree detector and an angle measurement trigger. The 0-degree detector is used for generating a signal when the supporting apparatus rotates to 0 degree so as to determine whether the current position is 0 degree. The 0-degree position is a position where a plane of the backup plate is parallel to a plane of the detector and is closest to the detector, i.e., a position where the back of the patient leans against the backup plate and the front of the patient faces the ray generator. The angle measurement trigger generates a signal at the image collection time to inform an angle measurement apparatus to measure an angle, is triggered by a high-voltage pulse signal rising edge in a pulsed exposure mode, and is triggered at an image collection interval by a timer in a continuous exposure mode. The angle measurement apparatus measures an angle of the current position relative to the 0-degree position when an angle measurement trigger signal is generated, and the calculation may be performed according to a rotation speed and a rotation time relative to the 0-degree position, and may also be achieved by an angle measurement instrument which includes, but is not limited to a potentiometer, a grating ruler and a gyroscope.

The correction apparatus is used for correcting an error between the rotation center and the angle. Markers with certain attenuation and obvious features convenient to recognize are used, are placed on the back of the bed board, and are uniformly distributed up and down, the markers have obvious features after x-ray imaging, then, the markers are detected by using an algorithm of a geometric correction module, the position of the rotation center and a rotation radius are calculated according to movement tracks of the markers, an angle error is corrected, and then, three-dimensional reconstruction is performed, by which more precise three-dimensional information may be obtained. The markers include, but are not limited to high-density objects, such as metal wires placed up and down within an imaging range and metal balls uniformly placed up and down. The markers may also be placed on a strip-shaped supporting object independent from the bed board and fixed to a turnplate;

a computer control system is used for implementing each step in embodiment 1, and includes a user interaction module, a control module, a geometric correction module, an image processing module and a data storage and management module.

The user interaction module is used for a user operation module, an information input module and a system state display module. The user operation module includes a starting switch, a rack movement control rod, a capturing starting point selection key and an imaging ending point selection key. The starting switch is used for starting and stopping data collection and may be achieved by an apparatus, such as a foot brake, a hand brake or a key. The movement control rod is used for controlling the up-and-down movement of the rack, and a user may moves, through the control rod, the detector and the ray generator to a position required to be captured. The imaging starting point selection key is used for selecting a starting point of large-scale imaging, the user controls the detector and the ray generator to move to a capturing starting point and presses down the starting point selection key, and at the moment, the positions of the detector and the ray generator are selected as capturing starting points. The imaging ending point selection key is used for selecting an ending point of large-scale imaging, the user controls the detector and the ray generator to move to a capturing ending point and presses down the ending point selection key, and at the moment, the positions of the detector and the ray generator are selected as capturing ending points. The information input module is used for inputting information, such as the name, age, gender, weight and height of a patient. The system state display module displays a working state of a system, and when the operation of the user does not satisfy requirements, relevant prompt information is displayed.

The control module includes a movement control module, an exposure control module and a collection control module. The movement control module controls the starting, stopping and rotating speeds of the patient supporting apparatus through instructions; controls the rack through instructions so that the detector and the ray generator synchronously move upwards or downwards; controls the size of a window of the beam limiter and the thickness of the filter through instructions; and calculates the number of segments for segmented collection and the movement distance of each segment according to a starting point and an ending point of a capturing range selected by the user.

The exposure control module sends an exposure condition set by the user to the high voltage generator, and may control the starting and stopping of an x-ray. A continuous exposure mode or a pulsed exposure mode may be selected as an exposure way.

The collection control module sends a synchronization signal to the detector while exposure, so that the image collection and x-ray collection of the detector are synchronously started and stopped.

The geometric correction module is used for correcting the position of the rotation center of the system and the measurement angle after scanning every time and compensating errors brought by assembling and positioning inaccuracy of the rotation module, in this way, the rotation module can be flexibly moved to support three-dimensional scanning imaging or two-dimensional capturing, which specifically includes the step for implementing angle correction in embodiment 1.

In conclusion, the present application provides a DR-based large-scale three-dimensional imaging method and discloses apparatus for carrying out the method of the invention. The multi-angle collection of a to-be-processed image may be achieved by the rotation of a rotation module, initial capturing angle data and rotation center calibration is performed on marker feature points in projection images captured from different angles in a to-be-processed image, and three-dimensional modeling is achieved according to the projection images and the calibrated initial capturing angle data and rotation center, wherein tangent points and the farthest distance point are acquired according to geometric features in an angle calibration process, and thus, the rotation center is marked through the three points to calibrate the measured initial angle data; at the same time, markers, such as metals, with obvious features under rays are additionally disposed, calibration may be conveniently performed in the formed to-be-processed image with the marker feature points as reference, and after the angle calibration is completed, segmented three-dimensional data is spliced by similarity calculation, so that three-dimensional images are spliced, and finally, a large-scale three-dimensional image is obtained. Projection data acquired by moving while rotating is further introduced to a movement position of a rack and is input as a parameter in a three-dimensional reconstruction process, and thus, the large-scale three-dimensional image is obtained at a time. For a corresponding apparatus, if it does not need to rotate, it is possible that a rotation motor is not started, and the apparatus may be used as an existing static DR; and the apparatus may achieve static DR, dynamic DR and three-dimensional DR functions at the same time, also solves the problem that the current three-dimensional DR can only achieve small-scale local three-dimensional imaging, and can scan the whole body and reconstruct a three-dimensional image when a patient is in a standing state.

Above descriptions are only embodiments of the present application, and are not intended to hence limit the patent scope of the present application. Any equivalent transformations made according to the contents of the description and the accompanying drawings of the present application are directly or indirectly applied to the related art and also fall within the patent protection scope of the present application, as defined by the appended set of claims, in a similar way.

## Claims

1. A DR (Digital Radiography)-based large-scale three-dimensional imaging method, comprising the steps:

   acquiring a to-be-processed image captured by a DR device, wherein the to-be-processed image comprises a plurality of projection images from different angles, and the projection images comprise marker feature points;

acquiring initial capturing angle data of each of the projection images;

calibrating coordinates of a rotation center and the initial capturing angle data according to the marker feature points so that feature angles of the marker feature points are consistent; and

performing three-dimensional reconstruction according to the projection images, the calibrated coordinates of the rotation center and the calibrated initial capturing angle data to obtain a reconstructed image, **characterized in that** the acquiring a to-be-processed image captured by a DR device comprises:

acquiring a starting point and an ending point of three-dimensional reconstruction, the maximum single-segment range that can be captured and reconstructed on a single segment by the DR device, and an overlapping range between two adjacent segments;

obtaining a total capturing range height according to the starting point, the ending point and the maximum single-segment range;

obtaining a total number of the segments and a movement distance of each of the segments according to the total capturing range height and the overlapping range;

obtaining an actual overlapping range according to the maximum single-segment range and the movement distance;

calculating a window height according to the actual overlapping range;

adjusting the DR device according to the window height, and setting a window width to be the maximum; wherein the window height is calculated along a direction in which the starting point and the ending point are connected; and

acquiring an exposure starting angle and an exposure ending angle, and completing the collection of each of the segments of the to-be-processed image according to the starting point, the exposure starting angle, the exposure ending angle, the movement distance and the ending point.

2. The DR-based large-scale three-dimensional imaging method according to claim 1, **characterized in that** the acquiring an exposure starting angle and an exposure ending angle, and completing the collection of each of the segments of the to-be-processed image according to the starting point, the exposure starting angle, the exposure ending angle, the movement distance and the ending point comprises:

starting exposure from the exposure starting angle at the starting point, rotating a rotation module of the DR device, and collecting the projection images in real time by the DR device;

when the rotation module of the DR device rotates to the exposure ending angle, stopping exposure and the collection of the to-be-processed image to complete the collection of the current segment of the to-be-processed image; and

moving a rack of the DR device to the ending point for the movement distance as a new starting point, and continuing to the collection of the next segment of the to-be-processed image until the ending point is reached.

3. The DR-based large-scale three-dimensional imaging method according to claim 1, **characterized in that** the calibrating coordinates of a rotation center and the initial capturing angle data according to the marker feature points comprises:

acquiring a central point O of a detector of the DR device, an X-ray source point S and initial capturing angle data $\theta_i$ of the $i^{th}$ projection image;

calculating a first distance SID between the central point of the detector and the X-ray source point;

establishing a two-dimensional rectangular coordinate system by taking a plane where the detector is located as an x axis and a line parallel to a straight line where a connecting line between the ray source point and the central point of the detector is located as a y axis; obtaining a projection position $(px_i, 0)$ of the marker feature point in the projection image on the plane where the detector is located;

acquiring the maximum projection position $(px_A, 0)$ of a first projection image with the maximum $px_i$ value, calculating corresponding first measurement angle data $\theta_A = \text{actan}(SID/px_A)$ according to the maximum projection position, acquiring first initial capturing angle data of the first projection image, and obtaining a first difference $d\theta_A$ of the first measurement angle data and the first initial capturing angle data;

acquiring the minimum projection position $(px_B, 0)$ of a second projection image with the minimum $px_i$ value, calculating corresponding second measurement angle data $\theta_B = \text{actan}(SID/px_B)$ according to the minimum projection position, acquiring second initial capturing angle data of the second projection image, and obtaining a second difference $d\theta_B$ of the second measurement angle data and the second initial capturing angle data;

calculating a third difference of x coordinates of two adjacent projection images, acquiring the maximum difference projection position $(px_D, 0)$ of the third difference and a corresponding third projection image, calculating corresponding third measurement angle data $\theta_D=actan(SID/px_D)$ according to the difference projection position, acquiring third initial capturing angle data of the corresponding third projection image, and obtaining a third difference $d\theta_D$ of the third measurement angle data and the third initial capturing angle data;

obtaining an average angle deviation $d\theta$ according to the first difference, the second difference and the third difference; and

correcting each piece of the initial capturing angle data $\theta_i$ according to the average angle deviation.

4. The DR-based large-scale three-dimensional imaging method according to claim 3, **characterized in that** the performing three-dimensional reconstruction according to the projection images, the calibrated coordinates of the rotation center and the calibrated initial capturing angle data to obtain a reconstructed image comprises:

inputting the projection images, calibrated initial capturing angles corresponding to the projection images and a calibrated rotation center to a reconstruction module to perform three-dimensional reconstruction to obtain the reconstructed image.

5. The DR-based large-scale three-dimensional imaging method according to claim 3, **characterized in that** the calibrating coordinates of a rotation center and the initial capturing angle data according to the marker feature points further comprises:

constructing a first expression $x_A=r*cos(\theta_A)+x_C$, $y_A=r*sin(\theta_A)+y_C$ of coordinates of a first origin A corresponding to the maximum projection position $(px_A, 0)$;

constructing a second expression $x_B=r*cos(\theta_B)+x_C$, $y_B=r*sin(\theta_B)+y_C$ of coordinates of a second origin B corresponding to the minimum projection position $(px_B, 0)$;

constructing a third expression $x_D=r*cos(\theta_D)+x_C$, $y_D=r*sin(\theta_D)+y_C$ of coordinates of a third origin D corresponding to the difference projection position $(px_D, 0)$;

acquiring a projection mapping formula $px*(SID-y)=x*SID$;

wherein px represents coordinates of a projected x axis, (x, y) represents coordinates of an origin before projection, and the origin comprises the first origin, the second origin and the third origin; and

seeking a solution according to the first expression, the second expression, the third expression and the projection mapping formula to obtain coordinates $(x_C, y_C)$ of the rotation center and a rotation radius r.

6. The DR-based large-scale three-dimensional imaging method according to claim 2, **characterized in that** the performing three-dimensional reconstruction according to the projection images, the calibrated coordinates of the rotation center and the calibrated initial capturing angle data to obtain a reconstructed image comprises:

performing three-dimensional reconstruction according to the projection image in each of the segments, the calibrated coordinates of the rotation center and the calibrated initial capturing angle data to obtain three-dimensional images;

traversing the three-dimensional images corresponding to the segments, when target three-dimensional images are traversed, acquiring intermediate tomographic images in the target three-dimensional images, acquiring marker feature points in the intermediate tomographic images, calculating the current correction angle of a connecting line between each marker feature point and the rotation center relative to the straight line where the connecting line between the ray source point and the central point of the detector is located, and comparing the current correction angle with a historical correction angle of a previous three-dimensional image, if the current correction angle is inconsistent with the historical correction angle, rotating the three-dimensional images so that the current correction angle is consistent with the historical correction angle; obtaining corrected target three-dimensional images; and

splicing all the corrected target three-dimensional images to obtain the reconstructed image.

7. The DR-based large-scale three-dimensional imaging method according to claim 6, **characterized in that** the splicing all the corrected target three-dimensional images to obtain the reconstructed image comprises:

traversing the segments, when target segments are traversed, acquiring the corrected target three-dimensional images corresponding to the target segments and a position (x0, y0, z0) of a historical comparison area of a previous target segment; searching the current comparison area with the highest similarity in target tomographic images of the corrected target three-dimensional images according to a content of the historical comparison area, and recording a position (x1, y1, z1) of the current comparison area;

calculating a deviation value $\Delta d=(x0, y0, z0)-(x1, y1, z1)$ of the current comparison area relative to the historical comparison area; and translating the corrected target three-dimensional images according to the deviation value;

and

acquiring the target tomographic images in the corrected target three-dimensional images, acquiring a new historical comparison area within an overlapping range of the corrected target three-dimensional images, and updating a position of the historical comparison area.

8. The DR-based large-scale three-dimensional imaging method according to claim 1, **characterized in that** after the adjusting the DR device according to the window height, and setting a window width to be the maximum, the DR-based large-scale three-dimensional imaging method comprises:

starting exposure from the exposure starting angle at the starting point, and rotating the rotation module of the DR device, at the same time, moving the DR device to the ending point, and collecting the projection images, the initial capturing angle data corresponding to the projection images and a movement position of the rack in real time by the DR device; and

when the DR device reaches the ending point, and the rotation module rotates to the exposure ending angle, stopping exposure and the collection of the to-be-processed image; and

the performing three-dimensional reconstruction according to the projection images, the calibrated coordinates of the rotation center and the calibrated initial capturing angle data to obtain a reconstructed image comprises: inputting the movement position of the rack, the projection images, calibrated initial capturing angles corresponding to the projection images and the calibrated coordinates of the rotation center to a reconstruction module to perform three-dimensional reconstruction to obtain the reconstructed image.

## Patentansprüche

1. Ein auf DR (Digitalradiographie) basierendes Verfahren zur großflächigen dreidimensionalen Bildgebung, das die folgenden Schritte umfasst:

Erfassen eines von einem DR-Gerät aufgenommenen, zu verarbeitenden Bildes, wobei das zu verarbeitende Bild eine Vielzahl von Projektionsbildern aus verschiedenen Winkeln umfasst und die Projektionsbilder Marker-Merkmalspunkte enthalten; Erfassen von Anfangsdaten zum Aufnahmewinkel jedes der Projektionsbilder; Kalibrieren der Koordinaten eines Rotationszentrums und der anfänglichen Aufnahmewinkeldaten entsprechend den Marker-Charakteristikpunkten, sodass die Charakteristikwinkel der Marker-Charakteristikpunkte übereinstimmen; und

Durchführen einer dreidimensionalen Rekonstruktion anhand der Projektionsbilder, der kalibrierten Koordinaten des Rotationszentrums und der kalibrierten anfänglichen Aufnahmewinkeldaten, um ein rekonstruiertes Bild zu erhalten, **dadurch gekennzeichnet, dass** das Erfassen eines von einem DR-Gerät aufgenommenen, zu verarbeitenden Bildes Folgendes umfasst:

Erfassen eines Startpunkts und eines Endpunkts der dreidimensionalen Rekonstruktion, des maximalen Einzelsegmentbereichs, der von der DR-Vorrichtung in einem einzelnen Segment aufgenommen und rekonstruiert werden kann, sowie eines Überlappungsbereichs zwischen zwei benachbarten Segmenten; Ermitteln einer Gesamthöhe des Aufnahmebereichs entsprechend dem Startpunkt, dem Endpunkt und dem maximalen Einzelsegmentbereich;

Ermitteln einer Gesamtzahl der Segmente und einer Bewegungsstrecke jedes der Segmente entsprechend der Gesamthöhe des Aufnahmebereichs und des Überlappungsbereichs;

Ermitteln eines tatsächlichen Überlappungsbereichs entsprechend dem maximalen Einzelsegmentbereich und der Bewegungsstrecke;

Berechnen einer Fensterhöhe entsprechend dem tatsächlichen Überlappungsbereich;

Anpassen des DR-Geräts entsprechend der Fensterhöhe und Einstellen der Fensterbreite auf den Maximalwert; wobei die Fensterhöhe entlang einer Richtung berechnet wird, in der der Startpunkt und der Endpunkt verbunden sind; und

Ermitteln eines Belichtungsstartwinkels und eines Belichtungsendwinkels sowie Abschließen der Erfassung jedes der Segmente des zu verarbeitenden Bildes entsprechend dem Startpunkt, dem Belichtungsstartwinkel, dem Belichtungsendwinkel, der Bewegungsstrecke und dem Endpunkt.

2. Das DR-basierte Verfahren zur großflächigen dreidimensionalen Bildgebung nach Anspruch 1, **dadurch gekennzeichnet, dass** das Erfassen eines Belichtungsstartwinkels und eines Belichtungsendwinkels sowie das Abschließen der Erfassung jedes Segments des zu verarbeitenden Bildes entsprechend dem Startpunkt, dem Belichtungsstartwinkel, dem Belichtungsendwinkel, der Bewegungsstrecke und dem Endpunkt Folgendes umfasst:

das Starten der Belichtung vom Belichtungsstartwinkel am Startpunkt, das Drehen eines Rotationsmoduls der DR-Vorrichtung und das Erfassen der Projektionsbilder in Echtzeit durch die DR-Vorrichtung;

wenn das Rotationsmodul der DR-Vorrichtung bis zum Belichtungsendwinkel gedreht ist, das Beenden der Belichtung und der Erfassung des zu verarbeitenden Bildes, um die Erfassung des aktuellen Segments des zu verarbeitenden Bildes abzuschließen; und

das Bewegen einer Zahnstange der DR-Vorrichtung um die Bewegungsstrecke zum Endpunkt als neuen Startpunkt und das Fortsetzen der Erfassung des nächsten Segments des zu verarbeitenden Bildes, bis der Endpunkt erreicht ist.

3. Das DR-basierte Verfahren zur großflächigen dreidimensionalen Bildgebung nach Anspruch 1, **dadurch gekennzeichnet, dass** die Kalibrierungskoordinaten eines Drehzentrums und die anfänglichen Aufnahmewinkeldaten gemäß den Marker-Merkmalspunkten Folgendes umfassen:

Erfassen eines Mittelpunktes O eines Detektors der DR-Vorrichtung, eines Röntgenquellenpunktes S und anfänglicher Aufnahmewinkeldaten $\theta_i$ des $i^{\text{ten}}$ Projektionsbildes;

Berechnung eines ersten Abstands SID zwischen dem Mittelpunkt des Detektors und dem Röntgenquellenpunkt;

Festlegung eines zweidimensionalen rechtwinkligen Koordinatensystems, indem eine Ebene, in der sich der Detektor befindet, als x-Achse und eine Gerade, die parallel zu einer Geraden verläuft, auf der sich eine Verbindungslinie zwischen dem Röntgenstrahlquellepunkt und dem Mittelpunkt des Detektors befindet, als y-Achse genommen wird; Ermittlung einer Projektionsposition $(px_i, 0)$ des Marker-Merkmalspunkts im Projektionsbild auf der Ebene, in der sich der Detektor befindet;

Ermitteln der maximalen Projektionsposition $(px_A, 0)$ eines ersten Projektionsbildes mit dem maximalen $px_i$-Wert, Berechnen entsprechender erster Messwinkeldaten $\theta_A = \text{actan}(SID/px_A)$ anhand der maximalen Projektionsposition, Ermitteln erster anfänglicher Aufnahmewinkeldaten des ersten Projektionsbildes und Ermitteln einer ersten Differenz $d\theta_A$ zwischen den ersten Messwinkeldaten und den ersten anfänglichen Aufnahmewinkeldaten;

Ermitteln der minimalen Projektionsposition $(px_B, 0)$ eines zweiten Projektionsbildes mit dem minimalen $px_i$-Wert, Berechnen der entsprechenden zweiten Messwinkeldaten $\theta_B = \text{actan}(SID/px_B)$ entsprechend der minimalen Projektionsposition, Ermitteln der zweiten anfänglichen Aufnahmewinkeldaten des zweiten Projektionsbildes und Ermitteln einer zweiten Differenz $d\theta_B$ zwischen den zweiten Messwinkeldaten und den zweiten anfänglichen Aufnahmewinkeldaten;

Berechnen einer dritten Differenz der x-Koordinaten zweier benachbarter Projektionsbilder, Ermitteln der Projektionsposition mit der maximalen Differenz $(px_D, 0)$ der dritten Differenz und eines entsprechenden dritten Projektionsbildes, Berechnen entsprechender dritter Messwinkeldaten $\theta_D = \text{actan}(SID/px_D)$ entsprechend der Projektionsposition mit der maximalen Differenz, Ermitteln dritter Anfangsaufnahmewinkeldaten des entsprechenden dritten Projektionsbildes und Ermitteln einer dritten Differenz $d\theta_D$ zwischen den dritten Messwinkeldaten und den dritten Anfangsaufnahmewinkeldaten;

Ermitteln einer durchschnittlichen Winkelabweichung $d\theta$ entsprechend der ersten Differenz, der zweiten Differenz und der dritten Differenz; und

Korrigieren jedes einzelnen Anfangsaufnahmewinkeldatensatzes $\theta_i$ entsprechend der durchschnittlichen Winkelabweichung.

4. Das DR-basierte Verfahren zur großflächigen dreidimensionalen Bildgebung gemäß Anspruch 3, **dadurch gekennzeichnet, dass** die Durchführung einer dreidimensionalen Rekonstruktion anhand der Projektionsbilder, der kalibrierten Koordinaten des Rotationszentrums und der kalibrierten anfänglichen Aufnahmewinkel-Daten zum Erhalten eines rekonstruierten Bildes umfasst:

Eingeben der Projektionsbilder, der den Projektionsbildern entsprechenden kalibrierten anfänglichen Aufnahmewinkel und eines kalibrierten Rotationszentrums in ein Rekonstruktionsmodul, um eine dreidimensionale Rekonstruktion durchzuführen und das rekonstruierte Bild zu erhalten.

5. Das DR-basierte Verfahren zur großflächigen dreidimensionalen Bildgebung gemäß Anspruch 3, **dadurch gekennzeichnet, dass** das Kalibrieren der Koordinaten eines Rotationszentrums und der anfänglichen Aufnahmewinkeldaten gemäß den Marker-Merkmalspunkten ferner umfasst:

das Erstellen eines ersten Ausdrucks $x_A = r^*\cos(\theta_A) + x_C$, $y_A = r^*\sin(\theta_A) + y_C$ für die Koordinaten eines ersten Ursprungs A, der der maximalen Projektionsposition $(px_A, 0)$ entspricht;

die Bildung eines zweiten Ausdrucks $x_B = r * \cos(\theta_B) + x_C$, $y_B = r * \sin(\theta_B) + y_C$ für die Koordinaten eines zweiten Ursprungs B, der der minimalen Projektionsposition ($px_B$, 0) entspricht;

Erstellen eines dritten Ausdrucks $x_D = r * \cos(\theta_D) + x_C$, $y_D = r * \sin(\theta_D) + y_C$ für die Koordinaten eines dritten Ursprungs D, der der Differenzprojektionsposition ($px_D$, 0) entspricht;

Ermitteln einer Projektionsabbildungsformel $px * (SID - y) = x * SID$;

wobei px die Koordinaten einer projizierten x-Achse darstellt, (x, y) die Koordinaten eines Ursprungs vor der Projektion darstellt und der Ursprung den ersten Ursprung, den zweiten Ursprung und den dritten Ursprung umfasst; und

Ermitteln einer Lösung gemäß dem ersten Ausdruck, dem zweiten Ausdruck, dem dritten Ausdruck und der Projektionsabbildungsformel, um die Koordinaten ($x_C$, $y_C$) des Drehzentrums und einen Drehradius r zu erhalten.

6. Das DR-basierte Verfahren zur großflächigen dreidimensionalen Bildgebung nach Anspruch 2, **dadurch gekennzeichnet, dass** die Durchführung einer dreidimensionalen Rekonstruktion anhand der Projektionsbilder, der kalibrierten Koordinaten des Rotationszentrums und der kalibrierten Anfangsdaten des Aufnahmewinkels zur Erzielung eines rekonstruierten Bildes Folgendes umfasst:

Durchführung einer dreidimensionalen Rekonstruktion anhand des Projektionsbildes in jedem der Segmente, der kalibrierten Koordinaten des Rotationszentrums und der kalibrierten Anfangsdaten des Aufnahmewinkels, um dreidimensionale Bilder zu erhalten;

Durchlaufen der den Segmenten entsprechenden dreidimensionalen Bilder; beim Durchlaufen der Ziel-3D-Bilder Erfassen von intermediären tomographischen Bildern in den Ziel-3D-Bildern; Erfassen von Marker-Charakteristikpunkten in den intermediären tomographischen Bildern; Berechnen des aktuellen Korrekturwinkels einer Verbindungslinie zwischen jedem Marker-Charakteristikpunkt und dem Rotationszentrum relativ zu der Geraden, auf der die Verbindungslinie zwischen dem Strahlungsquellenpunkt und dem Mittelpunkt des Detektors liegt; und Vergleichen des aktuellen Korrekturwinkels mit einem historischen Korrekturwinkel eines vorherigen dreidimensionalen Bildes; falls der aktuelle Korrekturwinkel nicht mit dem historischen Korrekturwinkel übereinstimmt, Drehen der dreidimensionalen Bilder, sodass der aktuelle Korrekturwinkel mit dem historischen Korrekturwinkel übereinstimmt; Erhalten korrigierter Ziel-3D-Bilder; und

Zusammenfügen aller korrigierten Ziel-3D-Bilder, um das rekonstruierte Bild zu erhalten.

7. Das DR-basierte Verfahren zur großflächigen dreidimensionalen Bildgebung nach Anspruch 6, **dadurch gekennzeichnet, dass** das Zusammenfügen aller korrigierten Ziel-3D-Bilder zum Erhalten des rekonstruierten Bildes umfasst:

das Abtasten der Segmente, wobei beim Abtasten von Zielsegmenten die den Zielsegmenten entsprechenden korrigierten Ziel-3D-Bilder sowie eine Position (x0, y0, z0) eines historischen Vergleichsbereichs eines vorherigen Zielsegments erfasst werden; Suchen des aktuellen Vergleichsbereichs mit der höchsten Ähnlichkeit in den Ziel-Tomographiebildern der korrigierten dreidimensionalen Zielbilder entsprechend einem Inhalt des historischen Vergleichsbereichs und Aufzeichnen einer Position (x1, y1, z1) des aktuellen Vergleichsbereichs; Berechnen eines Abweichungswerts $\Delta d = (x0, y0, z0) - (x1, y1, z1)$ des aktuellen Vergleichsbereichs relativ zum historischen Vergleichsbereich; und Verschieben der korrigierten dreidimensionalen Zielbilder entsprechend dem Abweichungswert; und

Erfassen der Ziel-Tomographiebilder in den korrigierten dreidimensionalen Zielbildern, Erfassen eines neuen historischen Vergleichsbereichs innerhalb eines Überlappungsbereichs der korrigierten dreidimensionalen Zielbilder und Aktualisieren einer Position des historischen Vergleichsbereichs.

8. Das DR-basierte Verfahren zur großflächigen dreidimensionalen Bildgebung nach Anspruch 1, **dadurch gekennzeichnet, dass** das DR-basierte Verfahren zur großflächigen dreidimensionalen Bildgebung nach dem Einstellen der DR-Vorrichtung entsprechend der Fensterhöhe und dem Einstellen einer Fensterbreite auf den Maximalwert Folgendes umfasst:

das Starten der Belichtung vom Belichtungsstartwinkel am Startpunkt aus und das Drehen des Rotationsmoduls der DR-Vorrichtung, wobei gleichzeitig die DR-Vorrichtung zum Endpunkt bewegt wird, sowie das Erfassen der Projektionsbilder, der den Projektionsbildern entsprechenden anfänglichen Aufnahmewinkeldaten und einer Bewegungsposition der Zahnstange in Echtzeit durch die DR-Vorrichtung; und

wenn die DR-Vorrichtung den Endpunkt erreicht und das Rotationsmodul in den Belichtungsendwinkel gedreht ist, das Beenden der Belichtung und der Erfassung des zu verarbeitenden Bildes; und

das Durchführen einer dreidimensionalen Rekonstruktion anhand der Projektionsbilder, der kalibrierten Koordinaten des Rotationszentrums und der kalibrierten Daten der anfänglichen Aufnahmewinkel, um ein rekonstruiertes Bild zu erhalten, umfasst:
Eingabe der Bewegungsposition des Gestells, der Projektionsbilder, der kalibrierten anfänglichen Aufnahmewinkel, die den Projektionsbildern entsprechen, und der kalibrierten Koordinaten des Drehzentrums in ein Rekonstruktionsmodul, um eine dreidimensionale Rekonstruktion durchzuführen und das rekonstruierte Bild zu erhalten.

**Revendications**

1. Procédé d'imagerie tridimensionnelle à grande échelle basé sur la radiographie numérique (DR), comprenant les étapes suivantes :

   acquisition d'une image à traiter capturée par un dispositif DR, dans lequel l'image à traiter comprend une pluralité d'images de projection prises sous différents angles, et les images de projection comprennent des points caractéristiques de repères ; acquisition des données d'angle de capture initiales de chacune des images de projection ;
   étalonnage des coordonnées d'un centre de rotation et des données d'angle de prise de vue initiales en fonction des points caractéristiques de repérage, de manière à ce que les angles caractéristiques de ces points soient cohérents ; et
   réalisation d'une reconstruction tridimensionnelle à partir des images de projection, des coordonnées étalonnées du centre de rotation et des données d'angle de prise de vue initiales étalonnées, afin d'obtenir une image reconstruite, **caractérisé en ce que** l'acquisition d'une image à traiter capturée par un dispositif DR comprend :

   l'acquisition d'un point de départ et d'un point d'arrivée de la reconstruction tridimensionnelle, de la portée maximale par segment pouvant être capturée et reconstruite sur un seul segment par le dispositif DR, et d'une plage de chevauchement entre deux segments adjacents ;
   l'obtention d'une hauteur totale de la plage de capture en fonction du point de départ, du point d'arrivée et de la portée maximale par segment ;
   l'obtention d'un nombre total de segments et d'une distance de déplacement de chacun des segments en fonction de la hauteur totale de la plage de capture et de la plage de chevauchement ;
   l'obtention d'une plage de chevauchement réelle en fonction de la plage maximale par segment et de la distance de déplacement ;
   le calcul d'une hauteur de fenêtre en fonction de la plage de chevauchement réelle ;
   ajuster le dispositif DR en fonction de la hauteur de la fenêtre, et régler la largeur de la fenêtre à sa valeur maximale ; la hauteur de la fenêtre étant calculée selon une direction reliant le point de départ et le point d'arrivée ; et
   acquérir un angle de début d'exposition et un angle de fin d'exposition, et achever la collecte de chacun des segments de l'image à traiter en fonction du point de départ, de l'angle de début d'exposition, de l'angle de fin d'exposition, de la distance de déplacement et du point d'arrivée.

2. Procédé d'imagerie tridimensionnelle à grande échelle basé sur la DR selon la revendication 1, **caractérisé en ce que** l'acquisition d'un angle de début d'exposition et d'un angle de fin d'exposition, et la réalisation de la collecte de chacun des segments de l'image à traiter en fonction du point de départ, de l'angle de début d'exposition, de l'angle de fin d'exposition, de la distance de déplacement et du point d'arrivée comprennent :

   le démarrage de l'exposition à partir de l'angle de début d'exposition au point de départ, la rotation d'un module de rotation du dispositif DR, et la collecte en temps réel des images de projection par le dispositif DR ;
   lorsque le module de rotation du dispositif DR atteint l'angle de fin d'exposition, l'arrêt de l'exposition et de la collecte de l'image à traiter afin de terminer la collecte du segment actuel de l'image à traiter ; et
   le déplacement d'une crémaillère du dispositif DR vers le point d'arrivée sur une distance de déplacement, ce point servant de nouveau point de départ, et la poursuite de la collecte du segment suivant de l'image à traiter jusqu'à ce que le point d'arrivée soit atteint.

3. Procédé d'imagerie tridimensionnelle à grande échelle basé sur la radiographie numérique (DR) selon la revendication 1, **caractérisé en ce que** les coordonnées d'étalonnage d'un centre de rotation et les données d'angle de capture initiales selon les points caractéristiques des marqueurs comprennent :

l'acquisition d'un point central O d'un détecteur du dispositif DR, d'un point source de rayons X S et des données d'angle de capture initiales $\theta_i$ de la $i^{ème}$ image de projection ;

le calcul d'une première distance SID entre le point central du détecteur et le point source de rayons X ;

l'établissement d'un système de coordonnées rectangulaires bidimensionnel en prenant comme axe x le plan dans lequel se trouve le détecteur et comme axe y une droite parallèle à la droite sur laquelle se trouve la ligne reliant le point source du faisceau et le point central du détecteur ; l'obtention d'une position de projection $(px_i, 0)$ du point caractéristique du marqueur dans l'image de projection sur le plan où se trouve le détecteur ;

acquérir la position de projection maximale $(px_A, 0)$ d'une première image de projection présentant la valeur $px_i$ maximale, calculer les premières données d'angle de mesure correspondantes $\theta_n = actan(SID/px_A)$ en fonction de la position de projection maximale, acquérir les premières données d'angle de capture initiales de la première image de projection, et obtenir une première différence $d\theta_A$ entre les premières données d'angle de mesure et les premières données d'angle de capture initiales ;

acquérir la position de projection minimale $(px_B, 0)$ d'une deuxième image de projection présentant la valeur $px_i$ minimale, calculer les deuxièmes données d'angle de mesure correspondantes $\theta_B = actan(SID/px_B)$ en fonction de la position de projection minimale, acquérir les deuxièmes données d'angle de capture initiales de la deuxième image de projection, et obtenir une deuxième différence $d\theta_B$ entre les deuxièmes données d'angle de mesure et les deuxièmes données d'angle de capture initiales ;

calculer une troisième différence entre les coordonnées x de deux images de projection adjacentes, déterminer la position de projection de la différence maximale $(px_D, 0)$ de la troisième différence et une troisième image de projection correspondante, calculer la troisième donnée d'angle de mesure correspondante $\theta_D = actan(SID/px_D)$ en fonction de la position de projection de la différence, déterminer la troisième donnée d'angle de capture initiale de la troisième image de projection correspondante, et obtenir une troisième différence $d\theta_D$ entre la troisième donnée d'angle de mesure et la troisième donnée d'angle de capture initiale ;

l'obtention d'un écart angulaire moyen $d\theta$ en fonction de la première différence, de la deuxième différence et de la troisième différence ; et

la correction de chaque donnée d'angle de capture initial $\theta_i$ en fonction de l'écart angulaire moyen.

4. Procédé d'imagerie tridimensionnelle à grande échelle basé sur la DR selon la revendication 3, **caractérisé en ce que** la reconstruction tridimensionnelle effectuée à partir des images de projection, des coordonnées calibrées du centre de rotation et des données d'angle de capture initiales calibrées pour obtenir une image reconstruite comprend :

l'entrée des images de projection, des angles de capture initiaux calibrés correspondant aux images de projection et d'un centre de rotation calibré dans un module de reconstruction afin d'effectuer une reconstruction tridimensionnelle pour obtenir l'image reconstruite.

5. Procédé d'imagerie tridimensionnelle à grande échelle basé sur la DR selon la revendication 3, **caractérisé en ce que** l'étalonnage des coordonnées d'un centre de rotation et des données d'angle de capture initial en fonction des points caractéristiques des marqueurs comprend en outre :

la construction d'une première expression $x_A = r*\cos(\theta_A) + x_C$, $y_A = r*\sin(\theta_A) + y_C$ des coordonnées d'une première origine A correspondant à la position de projection maximale $(px_A, 0)$ ;

la construction d'une deuxième expression $x_B = r*\cos(\theta_B) + x_C$, $y_B = r*\sin(\theta_B) + y_C$ des coordonnées d'une deuxième origine B correspondant à la position de projection minimale $(px_B, 0)$ ;

construire une troisième expression $x_D = r*\cos(\theta_D) + x_C$, $y_D = r*\sin(\theta_D) + y_C$ représentant les coordonnées d'une troisième origine D correspondant à la position de projection de la différence $(px_D, 0)$ ;

obtenir une formule de mise en correspondance de projection $px*(SID - y) = x*SID$ ;

où px représente les coordonnées d'un axe x projeté, $(x, y)$ représente les coordonnées d'une origine avant projection, et l'origine comprend la première origine, la deuxième origine et la troisième origine ; et

rechercher une solution selon la première expression, la deuxième expression, la troisième expression et la formule de mise en correspondance de projection afin d'obtenir les coordonnées $(x_C, y_C)$ du centre de rotation et un rayon de rotation r.

6. Procédé d'imagerie tridimensionnelle à grande échelle basé sur la DR selon la revendication 2, **caractérisé en ce que** la reconstruction tridimensionnelle effectuée à partir des images de projection, des coordonnées calibrées du centre de rotation et des données calibrées d'angle de capture initial pour obtenir une image reconstruite comprend :

la réalisation d'une reconstruction tridimensionnelle à partir de l'image de projection dans chacun des segments, des coordonnées calibrées du centre de rotation et des données calibrées d'angle de capture initial pour obtenir des images tridimensionnelles ;

parcourir les images tridimensionnelles correspondant aux segments ; lorsque les images tridimensionnelles cibles sont parcourues, acquérir des images tomographiques intermédiaires dans les images tridimensionnelles cibles, acquérir des points caractéristiques de repère dans les images tomographiques intermédiaires, calculer l'angle de correction actuel d'une ligne de jonction entre chaque point caractéristique de repère et le centre de rotation par rapport à la ligne droite sur laquelle se trouve la ligne de jonction entre le point source du rayon et le point central du détecteur, et comparer l'angle de correction actuel à un angle de correction historique d'une image tridimensionnelle précédente ; si l'angle de correction actuel est incompatible avec l'angle de correction historique, faire pivoter les images tridimensionnelles de manière à ce que l'angle de correction actuel soit compatible avec l'angle de correction historique ; obtenir des images tridimensionnelles cibles corrigées ; et assembler toutes les images tridimensionnelles cibles corrigées pour obtenir l'image reconstruite.

7. Procédé d'imagerie tridimensionnelle à grande échelle basé sur la radiographie numérique (DR) selon la revendication 6, **caractérisé en ce que** l'assemblage de toutes les images tridimensionnelles cibles corrigées pour obtenir l'image reconstruite comprend :

le balayage des segments ; lorsque des segments cibles sont balayés, l'acquisition des images tridimensionnelles cibles corrigées correspondant aux segments cibles et d'une position (x0, y0, z0) d'une zone de comparaison historique d'un segment cible précédent ; la recherche, parmi les images tomographiques cibles des images tridimensionnelles cibles corrigées, de la zone de comparaison actuelle présentant la plus grande similitude par rapport au contenu de la zone de comparaison historique, et l'enregistrement d'une position (x1, y1, z1) de la zone de comparaison actuelle ; le calcul d'une valeur d'écart $\Delta d = (x0, y0, z0) - (x1, y1, z1)$ de la zone de comparaison actuelle par rapport à la zone de comparaison historique ; et translatant les images tridimensionnelles cibles corrigées en fonction de la valeur d'écart ; et

acquérant les images tomographiques cibles dans les images tridimensionnelles cibles corrigées, acquérant une nouvelle zone de comparaison historique au sein d'une plage de chevauchement des images tridimensionnelles cibles corrigées, et mettant à jour une position de la zone de comparaison historique.

8. Procédé d'imagerie tridimensionnelle à grande échelle basé sur la radiographie numérique (DR) selon la revendication 1, **caractérisé en ce qu'**après avoir réglé le dispositif DR en fonction de la hauteur de la fenêtre et avoir défini la largeur de la fenêtre à sa valeur maximale, le procédé d'imagerie tridimensionnelle à grande échelle basé sur la radiographie numérique (DR) comprend :

le démarrage de l'exposition à partir de l'angle de début d'exposition au point de départ, et la rotation du module de rotation du dispositif DR, tout en déplaçant simultanément le dispositif DR vers le point d'arrivée, ainsi que la collecte en temps réel par le dispositif DR des images de projection, des données d'angle de capture initial correspondant aux images de projection et de la position de déplacement de la crémaillère ; et

lorsque le dispositif DR atteint le point final et que le module de rotation a atteint l'angle de fin d'exposition, l'arrêt de l'exposition et de la collecte de l'image à traiter ; et

la réalisation d'une reconstruction tridimensionnelle à partir des images de projection, des coordonnées calibrées du centre de rotation et des données calibrées d'angle de capture initial afin d'obtenir une image reconstruite, comprenant :

la saisie de la position de déplacement de la crémaillère, des images de projection, des angles de capture initiaux calibrés correspondant aux images de projection et des coordonnées calibrées du centre de rotation dans un module de reconstruction afin d'effectuer une reconstruction tridimensionnelle pour obtenir l'image reconstruite.

To obtain the image to be processed taken by the DR Device, the image to be processed comprises a plurality of projection images from different angles, and the projection image comprises a marker feature point; The initial shooting Angle data of each projection image is obtained.

The rotation center coordinates and the initial shooting Angle data are calibrated according to the marker feature points so that the feature angles of the marker feature points are consistent.

According to the projection image, the calibrated rotation center coordinates and the initial shooting Angle data, three-dimensional reconstruction is performed to obtain the reconstructed image.

Fig. 1

Fig. 2

Fig. 3

Fig. 4

Lateral orientation view of system

Rack module:
detector hanging arm

Rack module:
suspended slide rail

Rack module:
ray generator
hanging arm

High voltage
generator module

Backup
plate

Rotating shaft

X-ray
detector

Patient

Beam
Limiter

X-ray
generator

X-ray
detector

Beam
Limiter

X-ray
generator

X-ray
detector

Beam
Limiter

X-ray
generator

Patient supporting apparatus

Movable supporting apparatus

Computer control system

Control module

Image processing
module

User interaction
module

Data storage and
management module

Fig. 5

26

Rack module:
suspended slide rail

Rack module:
upright column
for supporting
detector

Rotating shaft

Rack module:
ray generator hanging arm

High voltage
generator module

X-ray
detector

Beam
limiter

X-ray
generator

Movement

Patient

Beam
limiter

X-ray
generator

Movement

Beam
limiter

X-ray
generator

Patient supporting apparatus

Movable supporting apparatus

Computer control system

Control module

Image processing
module

User interaction
module

Data storage and
management module

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOSSEMA FRANCIEN G et al.** Enabling 3D CT-scanning of cultural heritage objects using only in-house 2D X-ray equipment in museums. *NATURE COMMUNICATIONS*, 14 May 2024, vol. 15 (1)